# FASCICULE DE BREVET EUROPEEN

(11) **EP 2 515 838 B1**
(45) Date de publication et mention de la délivrance du brevet: **19.10.2016**
(21) Numéro de dépôt: 10807335.4
(22) Date de dépôt: 14.12.2010
(51) Int. Cl.: A61K 8/365, A61K 8/90, A61Q 19/00

(54) **COMPOSITION COSMETIQUE COMPRENANT UN DÉRIVÉ D'ACIDE JASMONIQUE**
KOSMETISCHE ZUBEREITUNG ENTHALTEND EIN JASMONSÄUREDERIVAT
COSMETIC COMPOSITION COMPRISING A JASMONIC ACID DERIVATIVE

(30) Priorité: 30.12.2009 US 290984 P; 22.12.2009 FR 0959356
(43) Date de publication de la demande: 31.10.2012
(73) Titulaire: L'Oréal, 75008 Paris (FR)
(72) Inventeur: KERMORVAN, Cécile, F-94000 Créteil (FR); SZESTAK, Magali, F-77350 Boissise Le Bertrand (FR)
(74) Mandataire: Hugodot, Yannick
(86) Numéro de dépôt international: PCT/FR2010/052711
(87) Numéro de publication internationale: WO 2011/083235

(56) Documents cités:
- EP-A2- 1 120 102
- EP-A2- 1 333 021
- WO-A2-2004/058151
- US-A1- 2005 063 925
- JONES C: "Multifunctional Synthetic Rheology Modifiers for Personal Care Formulations: More Than Just Thickeners", ROHM AND HAAS PERSONAL CARE,, 1 mai 2005 (2005-05-01), pages 1-23, XP007909943,

## Description

La présente invention concerne des compositions notamment cosmétiques et/ou dermatologiques comprenant un composé d'acide jasmonique et un polymère polyuréthane polyéther non ionique associatif, ainsi que l'utilisation de ces compositions dans un procédé de traitement des matières kératiniques d'êtres humains.
Plus particulièrement, les compositions de l'invention sont destinées au soin et/ou au maquillage des matières kératiniques.

Au sens de l'invention, on entend désigner par « matières kératiniques », par exemple, la peau, les muqueuses, les lèvres, le cuir chevelu, les cils, les sourcils et les cheveux.

Il est connu dans la demande EP-A-1333021 des composés d'acide jasmonique comme l'acide 3-hydroxy-2-pentyl-cyclopentane acétique pour favoriser la desquamation de la peau et stimuler le renouvellement épidermique, lutter contre les signes du vieillissement cutané, améliorer l'éclat du teint et/ou lisser la peau du visage. Dans la demande FR-A62921255 ces composés sont également décrits pour leur utilisation comme agent dépigmentant.

Malheureusement, l'introduction des composés d'acide jasmonique précédemment cités dans une formulation cosmétique aqueuse peut se traduire par une diminution non négligeable de la viscosité, induisant ainsi une fluidification importante de la composition.

Une composition trop fluide est difficile à appliquer sur les matières kératiniques. Une telle composition s'écoule des matières kératiniques, notamment de la peau, sur lesquelles elle est appliquée. Son application sur les matières kératiniques que l'on souhaite traiter manque de précision et rend ainsi son usage peu attractif.

En outre, la présence d'un composé d'acide jasmonique s'avère affecter le pouvoir épaississant de certains agents gélifiants conventionnels.

Les solutions alternatives consistant à suppléer à cette baisse de viscosité par l'ajout de cire(s) et/ou d'alcool gras ne s'avèrent pas satisfaisantes. Les formulations ainsi obtenues sont généralement trop épaisses et blanchissantes, difficiles à appliquer, et notamment à étaler, sur les matières kératiniques, et confèrent une sensation de lourdeur, de difficulté à pénétrer lors de l'application sur la peau.

Ainsi, il existe un besoin de disposer de composés d'acide jasmonique et qui puissent néanmoins être dotées d'un épaississement significatif si besoin est.

Il existe également un besoin de disposer de compositions cosmétiques ou dermatologiques présentant une viscosité appropriée à une application facile sur les matières kératiniques et notamment des compositions fluides à haute teneur en particules demeurant facilement vaporisables.

La présente invention a précisément pour objet de satisfaire à ces besoins.

Plus précisément, la présente invention concerne une composition, comprenant, dans un milieu physiologiquement acceptable contenant un milieu aqueux, au moins un composé d'acide jasmonique de formule (I) et au moins un polymère polyuréthane polyéther non ionique associatif.
La composition selon l'invention est en particulier une composition cosmétique et/ou pharmaceutique, notamment dermatologique.

De manière surprenante, les inventeurs ont observé que l'addition d'un composé d'acide jasmonique à une composition comprenant un polymère polyuréthane polyéther non ionique associatif n'affectait pas significativement la viscosité de ladite composition et permettait ainsi de formuler celle-ci sous une forme appropriée à sa manipulation lors de son application. En particulier, la viscosité de la composition ne chute pas.

Qui plus est, cette association spécifique composé d'acide jasmonique-polymère polyuréthane polyéther non ionique associatif permet également de conférer à une composition de l'invention un aspect agréable, et lors de son application, des propriétés de sensation confortable.

Selon encore un autre de ses objets, la présente invention concerne un procédé de traitement non thérapeutique de soin ou de maquillage des matières kératiniques comprenant l'application sur lesdites matières kératiniques d'une composition conforme à l'invention.

Le composé dérivé d'acide jasmonique est un composé choisi parmi ceux répondant à la formule (I) suivante : 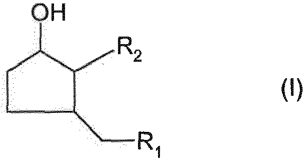 dans laquelle :
R₁ représente un radical COOR₃, R₃ désignant un atome d'hydrogène ou un radical alkyle en C₁-C₄ , éventuellement substitué par un ou plusieurs groupes hydroxyle ;
R₂ représente un radical hydrocarboné, saturé ou insaturé, linéaire ayant de 1 à 18 atomes de carbones, ou ramifié ou cyclique ayant de 3 à 18 atomes de carbone ; ainsi que leurs isomères optiques, et sels correspondants.

De préférence, R₁ désigne un radical choisi parmi -COOH, -COOMe, -COO-CH₂-CH₃, -COO-CH₂-CH(OH)-CH₂OH, -COOCH₂-CH₂-CH₂OH -COOCH₂-CH(OH)-CH₃. Préférentiellement, R₁ désigne un radical -COOH.

Préférentiellement, R₂ désigne un radical hydrocarboné, linéaire, saturé ou insaturé, et de préférence ayant de 2 à 7 atomes de carbone. En particulier, R₂ peut être un radical pentyl, pentenyl, hexyle, heptyle.

Selon un mode de réalisation, le composé de formule (I) est choisi parmi l'acide 3-hydroxy-2-[(2Z)-2-pentenyl]-cyclopentane acétique ou l'acide 3-hydroxy-2-pentyl-cyclopentane acétique. De préférence, le composé (I) est l'acide 3-hydroxy-2-pentyl-cyclopentane acétique ; ce composé peut être notamment sous la forme de sel de sodium.

Les sels des composés utilisables selon l'invention sont en particulier choisis parmi les sels de métal alcalin, par exemple sodium, potassium ; les sels de métal alcalino-terreux, par exemple calcium, magnésium, strontium, les sels métalliques, par exemple zinc, aluminium, manganèse, cuivre ; les sels d'ammonium de formule NH₄⁺ ; les sels d'ammonium quaternaires ; les sels d'amines organiques, comme par exemple les sels de méthylamine, de diméthylamine, de triméthylamine, de triéthylamine, d'éthylamine, de 2-hydroxyéthylamine, de bis-(2-hydroxyéthyl)amine, de la tri-(2-hydroxyéthyl)amine ; les sels de lysine, d'arginine. On utilise de préférence les sels choisis parmi les sels de sodium, potassium, magnésium, strontium, cuivre, manganèse, zinc. Préférentiellement, on utilise le sel de sodium.

Le composé de formule (I) défini précédemment peut être présent dans la composition selon l'invention en une teneur allant de 0,01 à 10 % en poids, par rapport au poids total de la composition, de préférence de 0,1 % à 5 % en poids.

La composition selon l'invention comprend un polymère polyuréthane polyéther associatif.

Au sens de la présente invention, on entend par "polymères associatifs" des polymères hydrophiles capables dans un milieu aqueux, de s'associer réversiblement entre eux ou avec d'autres molécules. Leur structure chimique comprend plus particulièrement au moins une zone hydrophile et au moins une zone hydrophobe.

Par "groupement hydrophobe", on entend un radical ou polymère à chaîne hydrocarbonée, saturée ou non, linéaire ou ramifiée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique ou bien d'un alcool gras polyalkyléné comme le stéareth-100. II peut également désigner un polymère hydrocarboné tel que par exemple le polybutadiène.

Les polyéthers polyuréthanes non ioniques selon l'invention comportent en général dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non ioniques comportent une liaison uréthane entre les séquences hydrophiles, d'où l'origine du nom.

Par extension figurent aussi parmi les polyéthers polyuréthanes non ioniques à chaîne hydrophobe, ceux dont les séquences hydrophiles sont liées aux séquences hydrophobes par d'autres liaisons chimiques.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

Le produit DW 1206B® de chez ROHM & HAAS à chaîne alkyle en C20 et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention peuvent aussi être choisis parmi ceux décrits dans l'article de G. Fonnum, J. Bakke et Fk. Hansen - Colloid Polym. Sci 271, 380.389 (1993).

Selon une forme particulière de l'invention, on utilisera un polyéther polyuréthane susceptible d'être obtenu par polycondensation d'au moins trois composés comprenant (i) au moins un polyéthylèneglycol comprenant de 150 à 180 moles d'oxyde d'éthylène, (ii) un alcool stéarylique polyoxyéthyléné comprenant 100 moles d'oxyde d'éthylène et (iii) un diisocyanate.
De tels polyéther polyuréthanes sont vendus notamment par la société ELEMENTIS sous l'appellation RHEOLATE FX 1100 ® qui est un polycondensat de polyéthylèneglycol à 136 moles d'oxyde d'éthylène, d'alcool stéarylique polyoxyéthyléné à 100 moles d'oxyde d'éthylène et de hexaméthylène diisocyanate (HDI) ayant poids moléculaire moyen en poids de 30000 (nom INCI : PEG-136/Steareth-1001/SMDI Copolymer).

Selon une autre forme particulière de l'invention on utilisera un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44®.

L'ACULYN 46® de nom INCI : PEG-150/Stearyl Alcohol/SMDI Copolymer est un polycondensat de polyéthylèneglycol à 150 ou 180 moles d'oxyde d'éthylène, d'alcool stéarylique et de méthylène bis(4-cyclohexyl-isocyanate) (SMDI) à 15% en poids dans une matrice de maltodextrine (4%) et d'eau (81%) (nom INCI : PEG-150/STEARYL ALCOHOL/SMDI COPOLYMER).

L'ACULYN 44® PEG-150/Decyl Alcohol/SMDI Copolymer est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%). (nom INCI : PEG-150/DECYL ALCOHOL/SMDI COPOLYMER).

Le polymère polyuréthane polyéther non ionique associatif décrit précédemment peut être présent dans la composition selon l'invention en une teneur allant de 0,05 % à 1 % en poids, par rapport au poids total de la composition, de préférence allant de 0,1 % à 0,6 % en poids, et préférentiellement allant de 0,1 % à 0,5 % en poids.

Avantageusement, le composé d'acide jasmonique de formule (I) et le polymère polyuréthane polyéther non ionique associatif décrits précédemment peuvent être présents dans la composition selon l'invention dans un rapport pondéral composé (I) /polymère polyuréthane polyéther non ionique associatif allant de 30 à 60, et de préférence allant de 40 à 50.

### PROTOCOLE DE MESURE DE LA VISCOSITE

La viscosité d'une composition de l'invention peut être mesurée selon tout procédé connu de l'homme de l'art, et notamment selon le procédé conventionnel suivant. Ainsi, la mesure peut être réalisée à 25 °C à l'aide d'un Contraves TV ou Rhéomat 180, équipé d'un mobile tournant à 200t/mn. L'homme du métier peut choisir le mobile permettant de mesure la viscosité, parmi les mobiles, M1 ou M2 sur la base de ses connaissances générales, de manière à pouvoir réaliser la mesure.
L'ajout d'un composé d'acide jasmonique tel que défini précédemment à une solution aqueuse comprenant le terpolymère selon l'invention se traduit par une variation de viscosité inférieure à 20% mesurée par rapport à la viscosité d'une solution comprenant uniquement le polymère, en particulier inférieure ou égale à 15%, en particulier inférieure ou égale à 10%.

La composition selon l'invention comprend un milieu aqueux physiologiquement acceptable.
Par « milieu physiologique acceptable », on entend désigner un milieu compatible avec les matières et/ou les fibres kératiniques d'êtres humains, comme par exemple, de manière non limitative, la peau, les muqueuses, les ongles, le cuir chevelu et/ou les cheveux.
Ce milieu aqueux physiologiquement acceptable comprend une phase aqueuse, éventuellement en mélange ou non avec un ou plusieurs solvants organiques tels qu'un alcool en C₁-C₈, notamment l'éthanol, l'isopropanol, le tert-butanol, le n-butanol, des polyols comme la glycérine, le propylène glycol, le butylèneglycol, et des éthers de polyol.

Une composition selon l'invention peut également comprendre une phase grasse, qui peut comprendre des huiles, des gommes, des cires usuellement utilisées dans le domaine d'application considéré.

Ainsi selon un mode de réalisation une composition selon l'invention peut en outre comprendre au moins une phase grasse choisie parmi une phase grasse solide à température ambiante (20-25 °C) et pression atmosphérique et/ou une phase grasse liquide à température ambiante (20-25 °C) et pression atmosphérique.

Une phase grasse liquide convenant à la mise en oeuvre de l'invention peut comprendre une huile volatile, une huile non volatile, et un mélange de celles-ci. Une huile volatile ou non volatile peut être une huile hydrocarbonée, notamment d'origine animale ou végétale, une huile synthétique, une huile siliconée, une huile fluorée ou un mélange de celles-ci.

Une phase grasse solide convenant à la mise en oeuvre de l'invention peut être, par exemple, choisie parmi les corps gras pâteux, les gommes, et leurs mélanges.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Lorsqu'une composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique.

Un émulsionnant et un coémulsionnant peuvent être présents dans une composition de l'invention en une proportion allant de 0,3 % à 30 % en poids, et en particulier de 0,5 à 20 % en poids par rapport au poids total de la composition.

Une émulsion selon l'invention peut, en outre, contenir des vésicules lipidiques.

Lorsqu'une composition selon l'invention est une solution ou un gel huileux, la phase grasse peut représenter plus de 90 % du poids total de la composition.

Une composition selon l'invention peut contenir également des adjuvants habituels dans le domaine considéré, tels que des tensioactifs, des émulsionnants, des gélifiants hydrophiles ou lipophiles, des additifs hydrophiles ou lipophiles, des conservateurs, des antioxydants, des solvants, des parfums, des charges, des filtres UVA et ou UVB (organique ou inorganique, solubles ou insolubles), des pigments, des fibres, des agents chélateurs, des absorbeurs d'odeur, des matières colorantes, et d'autres actifs cosmétiques ou pharmaceutiques.

Les quantités de ces différents adjuvants sont celles classiquement utilisées dans le domaine cosmétique, et peuvent être par exemple varier de 0,01 % à 30 % du poids total de la composition. De manière générale, les quantités sont ajustées en fonction de la formulation réalisée. Ces adjuvants, selon leur nature, peuvent être introduits dans la phase grasse, dans la phase aqueuse et/ou dans les sphérules lipidiques.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

Une composition de l'invention peut se présenter sous toutes les formes galéniques envisageables.
Notamment, une composition selon l'invention peut avoir la forme d'une solution aqueuse, hydroalcoolique; d'une dispersion; d'une émulsion eau-dans-huile, huile-dans-eau ou multiple; d'une suspension; de microcapsules ou microparticules; de dispersions vésiculaires de type ionique et/ou non ionique ; de composition pour aérosol comprenant également un agent propulseur sous pression. Préférentiellement, la composition selon l'invention peut être une émulsion huile-dans-eau.

Une composition selon l'invention peut se présenter sous la forme d'une composition pour soin capillaire, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.

Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage, composition de bronzage artificiel); une composition de maquillage du corps ou du visage telle qu'un fond de teint; une composition pour le bain; une composition désodorisante comprenant par exemple un agent bactéricide; une composition après-rasage; une composition épilatoire; une composition contre les piqûres d'insectes; une composition anti-douleur ; une composition dermatologique ou pharmaceutique pour traiter certaines maladies de la peau comme l'eczéma, la rosacée, le psoriasis, les lichens, les prurits sévères.

Lorsqu'une composition selon l'invention est destinée à un usage de type peeling, elle peut également se présenter sous toutes les formes galéniques évoquées ci-dessus pour autant qu'elle s'élimine facilement par rinçage, et notamment sous forme de gel aqueux, de solution aqueuse ou hydroalcoolique.

Une composition selon l'invention peut être appliquée par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux.

Une composition selon l'invention peut se présenter sous une forme fluide de type liquide vaporisable ou non, sous forme de pâte, d'émulsion directe ou inverse, ou de gel ou de support imprégné.

En particulier, une composition selon l'invention peut se présenter sous une forme solide, notamment compacte, pulvérulente ou coulée ou sous une forme de stick.

Une composition selon l'invention peut également se présenter sous la forme d'un produit de soin, d'un produit solaire ou après solaire, d'un produit de soin de photo-protection quotidienne, d'un produit pour le corps, d'un fond de teint à appliquer sur le visage ou sur le cou, d'un produit anti-cernes, d'un correcteur de teint, d'une crème teintée ou d'une base de maquillage pour le maquillage pour le visage ou d'une composition de maquillage pour le corps.

Une composition selon l'invention peut être mise en oeuvre à des fins d'amélioration de l'état général d'un épiderme, en particulier de la peau, et notamment pour le maintient ou la restauration de ses fonctions physiologiques et/ou de son aspect esthétique.

Ainsi, une composition selon l'invention peut être avantageusement mise en oeuvre afin de lutter contre le vieillissement de l'épiderme, maintenir et/ou stimuler l'hydratation et/ou lutter contre le dessèchement de la peau, améliorer la tonicité de la peau, maintenir ou restaurer la souplesse et l'élasticité de la peau, améliorer la minéralisation de l'épiderme, améliorer la vitalité de l'épiderme, faciliter les échanges inter-cellulaires, et lutter contre les gerçures et l'aspect craquelé de la peau.

Une composition selon l'invention peut être destinée à une application cosmétique et/ou dermatologique.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemples 1 à 4 comparatifs :

On a réalisé un gels aqueux avec 3 polymères épaississants asociatifs (2 polymères polyuréthane polyéther non ioniques associatifs selon l'invention et 1 polymère associatif hors invention), et pour chacun des polymères le gel été réalisé avec ou en l'absence de sel de sodium de l'acide 3-hydroxy-2-pentyl-cyclopentane acétique.

On a ensuite mesuré la viscosité des gels aqueux obtenus après 24 heures de stockage à température ambiante (viscosité mesurée à 25°C à l'aide d'un Contraves TV mobile M3 après 10 minutes de rotation à 200 tours/minutes.

| | **Exemple 1A** | **Exemple 1B (invention)** |
|---|---|---|
| Sel de sodium de l'acide 3-hydroxy-2-pentyl-cyclopentane acétique à 30 % dans un mélange eau/dipropylène glycol (70/30). | 0 | 3,3 % soit 1% MA |
| PEG-136/Steareth-100I/SMDI Copolymer (Rleolate FX1100 de chez Elementis) | 3 % | 3 % |
| Eau | Qsp 100 % | Qsp 100 % |
| Viscosité (pa.s) | 1,11 | 1,57 |

| | **Exemple 2A** | **Exemple 2B (invention)** |
|---|---|---|
| Sel de sodium de l'acide 3-hydroxy-2-pentyl-cyclopentane acétique à 30 % dans un mélange eau/dipropylène glycol (70/30) | 0 | 2,98 % soit 1% MA |
| PEG-150/DECYL ALCOHOUSMDI COPOLYMER (Aculyn 44 de chez Rohm & Haas) | 5% | 5% |
| Eau | Qsp 100 % | Qsp 100 % |
| Viscosité (pa.s) | 0,2 | 0,35 |

| | **Exemple 3A** | **Exemple 3B (hors invention)** |
|---|---|---|
| Sel de sodium de l'acide 3-hydroxy-2-pentyl-cyclopenta ne acétique à 30 % dans un mélange eau/dipropylène glycol (70/30) | 0 | 2,98 % soit 1% MA |
| AMMONIUM ACRYLOYLDIMETHYLTAURATE/ STEARETH-8 METHACRYLATE COPOLYMER (Aristoflex SNC de chez Clariant) | 2 % | 2 % |
| Eau | Qsp 100 % | Qsp 100 % |
| Viscosité (pa.s) | 7,1 | 5,2 |

| | **Exemple 4A** | **Exemple 4B (hors invention)** |
|---|---|---|
| Sel de sodium de l'acide 3-hydroxy-2-pentyl-cyclopentane acétique à 30 % dans un mélange eau/dipropylène glycol (70/30) | 0 | 2,98 % soit 1% MA |
| COPOLYMERE ACRYLAMIDE/ACRYLAMIDO 2-METHYL PROPANE SULFONATE DESODIUM EN EMULSION INVERSE A 40% DANS ISOPARAFFINE/EAU (Sépigel 305 de chez SEPPIC) | 3% | 3 % |
| Eau | Qsp 100 % | Qsp 100 % |
| Viscosité (pa.s) | 2,93 | 1,45 |

Ces essais montrent que seuls les gels comprenant les polyuréthane polyéther non ioniques associatifs Rheolate FX1100 et Aculyn 44 en présence du sel de sodium de l'acide 3-hydroxy-2-pentyl-cyclopentane acétique ne présentent pas de perte de viscosité. Alors que le gel comprenant l'Aristoflex SNC en présence du même actif présente une chute de viscosité d'environ 26 % et le gel comprenant le Sepigel 305 présente une chute de viscosité d'environ 50 %.
Ainsi, la présence de gomme de polyuréthane polyéther non ionique associatif permet de maintenir la viscosité du gel aqueux en présence du sel de sodium de l'acide 3-hydroxy-2-pentyl-cyclopentane acétique.

### Exemple 5 :

On a préparé une crème de soin du visage ayant la composition suivante :

| Composés | % poids |
|---|---|
| PEG-136/Steareth-100I/SMDI Copolymer (Rleolate FX1100 de chez Elementis) | 0,7 |
| Sel de sodium de l'acide 3-hydroxy-2-pentyl-cyclopentane acétique à 30 % dans un mélange eau/dipropylène glycol (70/30) | 3 % MA |
| Eau | Qsp |
| | 100 |
| sesquistéarate de méthyl glucose (Glucamate SS de chez Noveon) | 2 |
| sesquistéarate de méthyl glucose oxyéthyléné (Glucamate SSE 20 de chez Noveon) | 2 |
| Cyclohexasiloxane | 10 |
| Huile d'amandes d'abricot | 6 |
| Beurre de Shorea | 2 |
| Conservateur | 0,3 |

La composition obtenue à l'aspect d'une crème épaisse, fondante lors de son application sur la peau.
La composition appliquée sur le visage permet de raviver l'éclat du teint.

### Exemple 6 :

On prépare un fluide solaire vaporisable :

| COMPOSES | Ex A | Ex B |
|---|---|---|
| SEQUESTRANT | Qs | Qs |
| TRIETHANOLAMINE | Qs | Qs |
| BUTYLMETHOXYDtBENZYLMETHANE4-TERTIOBUTYL-4'-METHOXY-DIBENZOYLMETHANE (Parsol 1789 de DSM Nutritional product) | 1 | 3 |
| SALICYLATE de 2- ETHYLHEXYL (Néohéliopan OS de Symrise) | 0 | 5 |
| ETHYLHEXYL TRIAZONE (Uvinul T 150 de BASF) | 0,5 | 0,5 |
| TEREPHTHALYLIDENE DICAMPHOR SULFONIC ACID (Mexoryl SX de CHIMEX) | 1,5 | 1,5 |
| OCTOCRYLENE (Uvinul N 539 de BASF) | 10 | 10 |
| TITANIUM DIOXYDE (MT100 AQ de Tayca) | 0 | 3,5 |
| COPOLYMERE DIGLYCOL CYCLOHEXANEDIMETHANOL ISOPHTALATES SULFOISOPHTALATES (EASTMAN AQ 38S POLYMER de chez Eastman Chemical) | 2 | 2 |
| PEG-150/DECYL ALCOHOL/SMDI COPOLYMER (Aculyn 44 de chez Rohm & Haas) | 1 | 1,3 |
| CYCLOHEXASILOXANE | 5 | 5 |
| EAU | Qsp 100 | Qsp 100 |
| C₁₂-C₁₅ ALKYL BENZOATE (FINSOLV TN de chez FINETEX) | 6 | 1 |
| GLYCERINE | 3 | 3 |
| Sel de sodium de l'acide 3-hydroxy-2-pentyl-cyclopentane acétique à 30 % dans un mélange eau/dipropylène glycol (70/30) | 1 % MA | 1% MA |
| CONSERVATEUR | Qs | Qs |

Les produits obtenus sont stables, sprayables et confèrent à la peau une protection solaire en plus de l'activité biologique apportée par l'actif.

## Revendications

1. Composition comprenant, dans un milieu aqueux physiologiquement acceptable, un composé de formule (I) suivant : 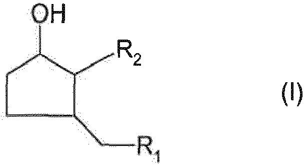 dans laquelle :
R₁ représente un radical COOR₃, R₃ désignant un atome d'hydrogène ou un radical alkyle en C₁-C₄ , éventuellement substitué par un ou plusieurs groupes hydroxyle ;
R₂ représente un radical hydrocarboné, saturé ou insaturé, linéaire ayant de 1 à 18 atomes de carbones, ou ramifié ou cyclique ayant de 3 à 18 atomes de carbone ;
ainsi que leurs isomères optiques, et sels correspondants ;
et un polymère polyuréthane polyéther non ionique associatif.

2. Composition selon la revendication 1, **caractérisé en ce que** le composé (I) est tel que R₁ désigne un radical choisi parmi -COOH, -COOMe, -COO-CH₂-CH₃, -COO-CH₂-CH(OH)-CH₂OH, -COOCH₂-CH₂-CH₂OH, -COOCH₂-CH(OH)-CH₃ ; R₂ désigne un radical hydrocarboné, linéaire, saturé ou insaturé, ayant de 2 à 6 atomes de carbone.

3. Composition selon la revendication 1 ou 2, **caractérisée en ce que** le composé (I) est l'acide 3-hydroxy 2-pentyl cyclopentane acétique.

4. Composition selon l'une quelconque des revendications précédentes, **caractérisée en ce que** le composé de formule (I) est présent en une teneur allant de 0,01 à 10 % en poids, par rapport au poids total de la composition, de préférence de 0,1 % à 5 % en poids.

5. Composition selon l'une quelconque des revendications précédentes, **caractérisée en ce que** le polyéther polyuréthane non ionique associatif comporte au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile.

6. Composition selon la revendication précédente, **caractérisée en ce que** le polyéther polyuréthane non ionique associatif est sous forme de tribloc.

7. Composition selon la revendication précédente, **caractérisée en ce que** le polyéther polyuréthane non ionique associatif est sous forme de tribloc dont la séquence hydrophile est une chaîne polyoxyéthylénée comportant de 50 à 1000 groupements oxyéthylénés.

8. Composition selon l'une quelconque des revendications précédentes, **caractérisée en ce que** le polyéther polyuréthane non ionique associatif est susceptible d'être obtenu par polycondensation d'au moins trois composés comprenant (i) au moins un polyéthylèneglycol comprenant de 150 à 180 moles d'oxyde d'éthylène, (ii) un alcool stéarylique polyoxyéthyléné comprenant 100 moles d'oxyde d'éthylène et (iii) un diisocyanate.

9. Composition selon l'une quelconque des revendications précédentes, **caractérisée en ce que** le polyéther polyuréthane non ionique associatif est le copolymère PEG-136/Steareth-100/SMDI.

10. Composition selon l'une quelconque des revendications 1 à 7, **caractérisée en ce que** le polyéther polyuréthane non ionique associatif est susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

11. Composition selon la revendication précédente, **caractérisée en ce que** le polyéther polyuréthane non ionique associatif est le copolymère PEG-150/Decyl Alcohol/SMDI ou le copolymère PEG-150/Stearyl Alcohol/SMDI.

12. Composition selon l'une quelconque des revendications précédentes, **caractérisée en ce que** le polymère polyuréthane polyéther non ionique associatif est présent en une teneur allant de 0,05 % à 1 % en poids, par rapport au poids total de la composition, de préférence allant de 0,1 % à 0,6 % en poids, et préférentiellement allant de 0,1 % à 0,5 % en poids.

13. Composition selon l'une quelconque des revendications précédentes, **caractérisée en ce qu'**elle se présente sous la forme d'une émulsion eau-dans-huile.

14. Procédé de traitement non thérapeutique des matières kératiniques comprenant l'application sur lesdites matières kératiniques d'une composition cosmétique telle que définie selon l'une quelconque des revendications précédentes.

## Patentansprüche

1. Zusammensetzung, die in einem physiologisch unbedenklichen wässrigen Medium eine Verbindung der folgenden Formel (I) 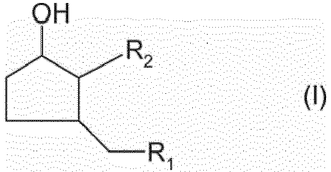 in der:
R₁ für einen COOR₃-Rest steht, wobei R₃ ein Wasserstoffatom oder einen C₁-C₄-Alkylrest, der gegebenenfalls durch einen oder mehrere Hydroxylgruppen substituiert ist, bedeutet;
R₂ für einen gesättigten oder ungesättigten Kohlenwasserstoffrest, der linear ist und 1 bis 18 Kohlenstoffatome aufweist oder verzweigt oder cyclisch ist und 3 bis 18 Kohlenstoffatome aufweist, steht;
sowie optische Isomere und entsprechende Salze davon
und ein assoziatives nichtionisches Polyurethanpolyetherpolymer
umfasst.

2. Zusammensetzung nach Anspruch 1, **dadurch gekennzeichnet, dass** die Verbindung (I) so beschaffen ist, dass R₁ für einen aus -COOH, -COOMe, -COO-CH₂-CH₃, -COO-CH₂-CH(OH)-CH₂OH, -COOCH₂-CH₂-CH₂OH und -COOCH₂-CH(OH)-CH₃ ausgewählten Rest steht und
R₂ für einen gesättigten oder ungesättigten linearen Kohlenwasserstoffrest mit 2 bis 6 Kohlenstoffatomen steht.

3. Zusammensetzung nach Anspruch 1 oder 2, **dadurch gekennzeichnet, dass** es sich bei der Verbindung (I) um 3-Hydroxy-2-pentylcyclopentanessigsäure handelt.

4. Zusammensetzung nach einem der vorhergehenden Ansprüche, **dadurch gekennzeichnet, dass** die Verbindung der Formel (I) in einem Gehalt im Bereich von 0,01 bis 10 Gew.-%, bezogen auf das Gesamtgewicht der Zusammensetzung, vorzugsweise von 0,1 bis 5 Gew.-%, vorliegt.

5. Zusammensetzung nach einem der vorhergehenden Ansprüche, **dadurch gekennzeichnet, dass** der assoziative nichtionische Polyurethanpolyether mindestens zwei lipophile Kohlenwasserstoffketten mit 6 bis 30 Kohlenstoffatomen aufweist, die durch einen hydrophilen Block getrennt sind, wobei es sich bei den Kohlenwasserstoffketten um seitenständige Ketten oder Ketten am Ende des hydrophilen Blocks handeln kann.

6. Zusammensetzung nach dem vorhergehenden Anspruch, **dadurch gekennzeichnet, dass** der assoziative nichtionische Polyurethanpolyether Dreiblockform aufweist.

7. Zusammensetzung nach dem vorhergehenden Anspruch, **dadurch gekennzeichnet, dass** der assoziative nichtionische Polyurethanpolyether Dreiblockform aufweist, wobei es sich bei dem hydrophilen Block um eine Polyoxyethylenkette mit 50 bis 1000 Oxyethylengruppen handelt.

8. Zusammensetzung nach einem der vorhergehenden Ansprüche, **dadurch gekennzeichnet, dass** der assoziative nichtionische Polyurethanpolyether durch Polykondensation von mindestens drei Verbindungen, umfassend (i) mindestens ein Polyethylenglykol mit 150 bis 180 mol Ethylenoxid, (ii) einen polyoxyethylenierten Stearylalkohol mit 100 mol Ethylenoxid und (iii) ein Diisocyanat, erhältlich ist.

9. Zusammensetzung nach einem der vorhergehenden Ansprüche, **dadurch gekennzeichnet, dass** es sich bei dem assoziativen nichtionischen Polyurethanpolyether um das PEG-136/Steareth-100/SMDI Copolymer handelt.

10. Zusammensetzung nach einem der Ansprüche 1 bis 7, **dadurch gekennzeichnet, dass** der assoziative nichtionische Polyurethanpolyether durch Polykondensation von mindestens drei Verbindungen, umfassend (i) mindestens ein Polyethylenglykol mit 150 bis 180 mol Ethylenoxid, (ii) Stearylalkohol oder Decylalkohol und (iii) mindestens ein Diisocyanat, erhältlich ist.

11. Zusammensetzung nach einem der vorhergehenden Ansprüche, **dadurch gekennzeichnet, dass** es sich bei dem assoziativen nichtionischen Polyurethanpolyether um das PEG-150/Decyl Alcohol/SMDI Copolymer oder das PEG-150/Stearyl Alcohol/SMDI Copolymer handelt.

12. Zusammensetzung nach einem der vorhergehenden Ansprüche, **dadurch gekennzeichnet, dass** das assoziative nichtionische Polyurethanpolyetherpolymer in einem Gehalt im Bereich von 0,05 bis 1 Gew.-%, bezogen auf das Gesamtgewicht der Zusammensetzung, vorzugsweise im Bereich von 0,1 bis 0,6 Gew.-% und bevorzugt im Bereich von 0,1 bis 0,5 Gew.-% vorliegt.

13. Zusammensetzung nach einem der vorhergehenden Ansprüche, **dadurch gekennzeichnet, dass** sie in Form einer Wasser-in-Öl-Emulsion vorliegt.

14. Verfahren zur nichttherapeutischen Behandlung von Keratinfasern, bei dem man auf die Keratinfasern eine kosmetische Zusammensetzung gemäß einem der vorhergehenden Ansprüche aufbringt.

## Claims

1. Composition comprising, in a physiologically acceptable aqueous medium, a compound of following formula (I): 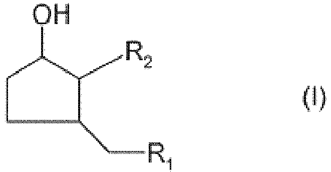 in which:
R₁ represents a COOR₃ radical, R₃ denoting a hydrogen atom or a C₁-C₄ alkyl radical optionally substituted with one or more hydroxyl groups;
R₂ represents a saturated or unsaturated, linear having from 1 to 18 carbon atoms, or branched or cyclic having from 3 to 18 carbon atoms, hydrocarbon-based radical;
and also the optical isomers and corresponding salts thereof;
and a nonionic associative polyurethane polyether polymer.

2. Composition according to Claim 1, **characterized in that** the compound (I) is such that R₁ denotes a radical chosen from -COOH, -COOMe, -COO-CH₂-CH₃, -COO-CH₂-CH (OH) -CH₂OH, -COOCH₂-CH₂-CH₂OH, -COOCH₂-CH(OH)-CH₃;
R₂ denotes a linear, saturated or unsaturated, hydrocarbon-based radical having from 2 to 6 carbon atoms.

3. Composition according to Claim 1 or 2, **characterized in that** the compound (I) is 3-hydroxy-2-pentylcyclopentaneacetic acid.

4. Composition according to any one of the preceding claims, **characterized in that** the compound of formula (I) is present in a content ranging from 0.01 to 10% by weight and preferably from 0.1 to 5% by weight relative to the total weight of the composition.

5. Composition according to any one of the preceding claims, **characterized in that** the nonionic associative polyurethane polyether comprises at least two lipophilic hydrocarbon-based chains having from 6 to 30 carbon atoms, separated by a hydrophilic block, the hydrocarbon-based chains possibly being pendant chains or chains at the end of the hydrophilic block.

6. Composition according to the preceding claim, **characterized in that** the nonionic associative polyurethane polyether is in triblock form.

7. Composition according to the preceding claim, **characterized in that** the nonionic associative polyurethane polyether is in triblock form, the hydrophilic block of which is a polyoxyethylene chain comprising from 50 to 1000 oxyethylene groups.

8. Composition according to any one of the preceding claims, **characterized in that** the nonionic associative polyurethane polyether is obtainable by polycondensaton of at least three compounds comprising (i) at least one polyethylene glycol comprising from 150 to 180 mol of ethylene oxide, (ii) a polyoxyethylenated stearyl alcohol comprising 100 mol of ethylene oxide, and (iii) a diisocyanate.

9. Composition according to any one of the preceding claims, **characterized in that** the nonionic associative polyurethane polyether is the PEG-136/Steareth-100/SMDI copolymer.

10. Composition according to any one of Claims 1 to 7, **characterized in that** the nonionic associative polyurethane polyether is obtainable by polycondensaton of at least three compounds comprising (i) at least one polyethylene glycol comprising from 150 to 180 mol of ethylene oxide, (ii) stearyl alcohol or decyl alcohol, and (iii) at least one diisocyanate.

11. Composition according to the preceding claim, **characterized in that** the nonionic associative polyurethane polyether is the PEG-150/Decyl Alcohol/SMDI copolymer or the PEG-150/Stearyl Alcohol/SMDI copolymer.

12. Composition according to any one of the preceding claims, **characterized in that** the nonionic associative polyurethane polyether polymer is present in a content ranging from 0.05 to 1% by weight, preferably ranging from 0.1 to 0.6% by weight and preferentially ranging from 0.1 to 0.5% by weight relative to the total weight of the composition.

13. Composition according to any one of the preceding claims, **characterized in that** it is in the form of a water-in-oil emulsion.

14. Method for the non-therapeutic treatment of keratin materials, comprising the application, to said keratin materials, of a cosmetic composition as defined in any one of the preceding claims.
